Europäisches Patentamt

European Patent Office

Office européen des brevets

Numéro de publication: **0 367 637**
**A1**

## DEMANDE DE BREVET EUROPEEN

Numéro de dépôt: **89402313.4**

Int. Cl.5 **C12P 19/34 , C12N 15/10**

Date de dépôt: **21.08.89**

Priorité: **26.08.88 FR 8811263**

Date de publication de la demande:
**09.05.90 Bulletin 90/19**

Etats contractants désignés:
**AT BE CH DE ES FR GB GR IT LI LU NL SE**

Demandeur: **CENTRE NATIONAL DE LA RECHERCHE SCIENTIFIQUE**
**15, quai Anatole France**
**F-75007 Paris(FR)**

Inventeur: **Mechali, Marcel**
**71 Rue Emile Goeury**
**F-94800 Villejuif(FR)**

Mandataire: **Ores, Irène et al**
**CABINET ORES 6, Avenue de Messine**
**F-75008 Paris(FR)**

Nouveau système eucaryote multifonctionnel in vitro et ses applications.

Nouveau système eucaryote multifonctionnel in vitro apte à permettre à lui seul la réalisation in vitro d'importantes réactions utilisées en biologie moléculaire et en génie génétique, caractérisé en ce qu'il est constitué par un extrait de cellules germinales de Xénope.

Procédé de préparation dudit extrait.

Application de cet extrait à la synthèse d'acides nucléiques et de protéines, à la ligation d'ADN, à la mutagénèse dirigée in vitro, etc...

EP 0 367 637 A1

## NOUVEAU SYSTEME EUCARYOTE MULTIFONCTIONNEL IN VITRO ET SES APPLICATIONS

La présente invention a été faite à l'Institut Jacques MONOD - Université de PARIS VII - Unité associée au CENTRE NATIONAL DE LA RECHERCHE SCIENTIFIQUE N. UM 3. Elle est relative à un nouveau système eucaryote multifonctionnel in vitro constitué par des extraits de cellules germinales de Xénope et à leurs applications notamment pour la réalisation de mutagénèses in vitro, la synthèse d'acides nucléiques, la ligation de l'ADN et la synthèse de protéines in vitro.

Dès 1969 [GURDON JR, BIRNSTEIL ML, SPEIGHT VA, BIOCHIM. BIOPHYS. ACTA 174 - (1969), p. 614-618] on avait observé la réplication in vivo d'ADN purifié introduit par micro-injection dans du cytoplasme d'oeufs de Xénope et dès 1978 [BENBOW et Al. CELL, 13 (1978)] p. 307-318 on a proposé des systèmes acellulaires dérivés d'oeufs de Xénope pour étudier la réplication de l'ADN, cependant avec des résultats non concluants puisqu'ils n'ont pas permis de faire la distinction entre la synthèse réparatrice d'ADN et la replication semi-conservatrice. ATTARDI et Al. [NATURE 270 (1977) p. 754-756] et BENBOW & Al. [CELL, 12 (1977) p. 191-204] ont également montré que la recombinaison est favorisée par les extraits d'ovocytes ou d'oeufs.

M. MECHALI & R.M. HARLAND (CELL, Vol. 30, p. 93-101, Août 1982) ont décrit un système eucaryote dérivé d'oeufs de Xénopus laevis, qui convertit in-vitro de l'ADN circulaire monocaténaire en la forme double-brin complète fermée par covalence, dans le cadre d'une réaction ribonucléoside-triphosphate-dépendante. Ils ont décrit, dans cet Article, la conservation et la ligation d'ADN par des homogenats d'oeufs de Xénope, ainsi que la formation en présence de ces derniers, d'ADN super-rhélicoïdal et l'aptitude desdits extraits d'oeufs de Xénope à favoriser la synthèse d'ADN in vitro. Cet Article indique cependant que si la synthèse du brin complémentaire est commencée de façon efficace sur l'ADN monocaténaire, par contre il n'a pas été observé de replication avec une matrice d'ADN double-brin. De plus, M. ALMOUZNI et M. MECHALI[THE EMBO JOURNAL, VOL. 7, n° 3, p. 665-672 (1988)] ont montré que cette replication est couplée à l'assemblage en chromatine de l'ADN en cours de replication. La replication de l'ADN double-brin a également été observée par BLOW et LASKEY [ CELL, 47 (1986) p. 577-587] dans des extraits centrifugés à basse vitesse, mais avec des rendements très faibles.

Les traceurs radio-actifs sont d'utilisation routinière dans la recherche fondamentale et appliquée. La recherche de gènes et de leurs expressions nécessite de tels traceurs permettant de les identifier de façon spécifique. Ainsi, pour détecter et isoler une séquence génétique spécifique, on essaie de l'hybrider avec une séquence complémentaire que l'on a préalablement rendue radio-active afin de faciliter son repérage. C'est une telle opération qui est faite dans les expériences de SOUTHERN BLOT permettant d'identifier des gènes spécifiques au niveau d'un tissu ou d'un organisme donné. De même, lorsque l'on recherche l'expression d'un gène donné enARN, on détecte cet ARN grâce à une séquence complémentaire d'ADN radio-active.

Différentes méthodes de fabrication de ces séquences ou matrices radio-actives ont été utilisées dont la nick-translation (RIGBY & AL., J. MOL. BIOL. (1977) 113, p. 237), le random-priming (FEINBERG 7 VOGELSTEIN. ANAL. BIOCH. (1983) 132, p. 6-13). Ces méthodes utilisent dans tous les cas des DNA polymérases bactériennes purifiées, le plus souvent la DNA polymérase 1 d'E. COLI (nick translation) ou la DNA polymérase 1 d'E. COLI fragment KLENOW (random-priming), nécessitent l'addition de DNase 1 (pour la nick translation), l'addition des 4 désoxynucléotides, et l'addition d'un tampon contenant principalement du Tris, du $MgCl_2$, et du NaCl.

Ces méthodes donnent satisfaction mais requièrent l'intervention de réactifs multiples et de nombreuses manipulations.

Par ailleurs, la ligation de l'ADN in vitro est la base même du génie génétique. Chaque fois que l'on cherche à cloner un gène ou à l'analyser, il faut l'insérer dans un vecteur recombinant. Cette opération n'est possible qu'en effectuant des ligations de cette séquence avec le vecteur hôte. La ligation est une opération qui permet également la formation de l'ADN super-hélicoïdal à partir d'ADN circulaire ouvert. Il est très fréquent de nécessiter un ADN plasmidique ou de phage sous sa forme native, c'est-à-dire super-hélicoïdal. Or, après purification, l'ADN est sujet à des coupures simple-brin qui le transforment en ADN circulaire après un temps de stockage variable. Cette transformation est extrêmement accélérée si l'ADN est radio-actif, en raison de la radiolyse. La ligation requiert l'intervention de l'ADN ligase, le plus souvent l'ADN ligase purifiée de cellules infectées par le bactériophage T4, d'ATP et d'un tampon, la multiplicité des réactifs mis en oeuvre impliquant de nombreuses manipulations.

L'isolement et la caractérisation des gènes a amené à rechercher les relations entre la structure du gène et sa régulation ou son expression. De même, la séquence d'une protéine étant déduite de la séquence d'ARN, de très nombreuses recherches visant à modifier les protéines, et donc leurs activités, ont été menées aussi bien sur le plan fondamental que sur le plan des applications.

Ces études nécessitaient la production systématique de mutants du gène considéré. L'altération de la séquence d'ADN par mutagénèse in-vitro est maintenant devenue un moyen très utilisé pour analyser les rapports entre structure et fonction de l'ADN et les protéines pour lesquelles il code (SHORTLE et al., ANN. REV. GENET., 1980, 15, p. 265 ; SMITH, ANN. REV. GENET., 1985, 19, p. 423).

De façon idéale, les altérations doivent être produites avec une efficacité maximum et avec un minimum de manipulations, car il est nécessaire dans ce type de recherches d'obtenir un très grand nombre de mutants différents. Un certain nombre de techniques ont déjà été décrites à ce jour qui, dans tous les cas, font appel à plusieurs enzymes procaryotes et nécessitent une certaine expertise au niveau des différentes manipulations de ces enzymes (ZOLLER et SMITH, METHODS IN ENZYMOLOGY (1983) 100, p. 468-500). Les systèmes classiques proposés actuellement utilisent, pour obtenir de l'ADN muté, la DNA-polymerase de KLENOW et la DNA-ligase, les quatre désoxynucléotides triphosphates, l'ATP et un tampon conte-nant du Tris, du $MgCl_2$, du NaCl, du 2-mercaptoéthanol (ZOLLER et SMITH, Loc. cité), c'est-à-dire de nombreux réactifs et des manipulations multiples.

La synthèse d'ARN in-vitro s'avère nécessaire pour différentes opérations classiques en biologie moléculaire fondamentale et appliquée, comme la fabrication de traceurs ARN radio-actifs, la fabrication de protéines in-vitro, la recherche de signaux spécifiques de régulation portés par la séquence ARN.

L'étude de l'activité génique implique de pouvoir disposer de protéines codées par les gènes, afin d'en étudier leur fonction. La synthèse de protéines en grande quantité s'effectue le plus souvent par l'intermédiaire de vecteurs d'expression recombinants exprimant la protéine généralement chez la bactérie et quelquefois dans la cellule eukaryote. Cette méthode nécessite plusieurs manipulations dont le clonage de la séquence codante du gène, sa fusion en phase avec des promoteurs s'exprimant chez la bactérie ou la cellule considérée, et la protéine obtenue est le plus souvent hybride. Enfin les protéines obtenues dans la majorité de ces systèmes sont peu solubles et souvent toxiques pour la bactérie ou cellule hôte productrice. La synthèse de protéines in vitro à partir des ARNs codant pour ces protéines présente de très nombreux avantages : pas de transformation à effectuer ; pas de culture cellulaire ou bactérienne en masse à entreprendre ; toxicité très faible car les événements cellulaires de transcription, replication ou division cellulaire ne sont pas requis ; très grande souplesse d'utilisation caractéristique des systèmes in vitro. Ce type de système de traduction in vitro des protéines est actuellement réalisé soit à l'aide d'extraits de réticulocytes de Lapin, soit à l'aide d'extraits de germes de blé (références.....).

Il ressort de la rapide revue qui précède que chacune des réactions fondamentales en biologie moléculaire dont il vient d'être fait mention nécessite l'utilisation de plusieurs réactifs et de nombreuses manipulations.

La possibilité de disposer d'un seul système eucaryote permettant de réaliser in vitro les plus importantes réactions utilisées en biologie moléculaire et en génie génétique, telles que la synthèse d'acides nucléiques, la synthèse de protéines, la ligation de l'ADN et la mutagénèse dirigée in vitro, constituerait un progrès important dans ce domaine.

C'est ce progrès qu'est apte à procurer un système eucaryote constituée par un extrait de cellules germinales de Xénope, qui permet de réaliser totalement in vitro et en un minimum d'étapes, à la fois la synthèse de matrices d'ADN ou d'ARN, la ligation de l'ADN, la mutagénèse dirigée in vitro et la synthèse de protéines in vitro. Un tel extrait contient de façon active in vitro tous les composants ioniques et non-ioniques nécessaires à la réalisation de l'ensemble de ces réactions, en sorte qu'il constitue à lui seul un kit complet utilisable dans des réactions biologiques très différentes, avec une grande efficacité et une grande simplicité d'utilisation. Ce type de système multifonctionnel in vitro est actuellement unique. L'extrait de cellules germinales de Xénope est d'obtention facile .et il est stable pendant au moins 6 mois à - 80° C sans perte d'activité et à partir de la ponte d'un seul animal on peut obtenir jusqu'à environ 10 ml d'extrait d'oeufs de Xénope.

Il présente en outre l'avantage de constituer le premier système eucaryote permettant de réaliser une mutagénèse in vitro, seuls des systèmes bactériens procaryotes, ayant été proposés à ce jour.

La présente invention a pour objet un nouveau système eucaryote multifonctionnel in vitro, apte à permettre à lui seul la réalisation in vitro d'importantes réactions utilisées en biologie moléculaire et en génie génétique, notamment la synthèse d'acides mucléiques, la synthèse de protéines, la ligation de l'ADN, la mutagénèse dirigée in vitro, le séquençage d'ADN, lequel système eucaryote est caractérisé en ce qu'il est constitué par un extrait de cellules germinales de Xénope obtenu à la suite d'au moins une centrifugation à 0-1° C à 14 000 g pendant 20 minutes, éventuellement suivie(s) d'une centrifugation à 140 000 g pendant 45 minutes à 0-1˚ C et ou éventuellement précédée d'une centrifugation de brève durée à faible vitesse de l'ordre de 30 à 150 g, pendant 40 à 60 secondes.

La présente invention a également pour objet un procédé de production d'extraits d'oeufs de Xénope à partir d'oeufs récoltés résultant de la ponte de Xénopes femelles préalablement traitées par de l'hormone chorionique gonadotrope, caractérisé en ce que les oeufs, préalablement dégélifiés et rincés par un tampon d'extraction approprié, sont soumis à une à deux centrifugations à 0-1˚ C, à 14 000 g pendant 20 minutes à la suite de laquelle ou desquelles l'extrait obtenu est recueilli et stocké à basse température (congélation à -80˚ C ou sous azote liquide), cette ou ces centrifugation(s) étant éventuellement suivie(s) d'une centrifugation de l'extrait collecté au-dessus du culot de la ou des première(s) centrifugations(s), à 140 000 g pendant 45 minutes environ à 0-1˚ C environ et/ou étant éventuellement précédée-(s) d'une centrifugation de brève durée à faible vitesse, de l'ordre de 30 à 150 g pendant 40 à 60 secondes, avantageusement dans une huile neutre de densité intermédiaire entre l'oeuf et l'eau, destinée à éliminer le tampon d'extraction des oeufs.

La présente invention a en outre pour objet un procédé de réalisation de plusieurs réactions fondamentales en biologie moléculaire à l'aide d'un seul et unique système multifonctionnel eucaryote, notamment de réalisation de la synthèse d'acides nucléiques, de la synthèse de protéines, de la ligation de l'ADN, de la mutagénèse dirigée in-vitro, du séquençage d'ADN, caractérisé en ce qu'on incube un acide nucléique de départ avec de l'extrait de cellules germinales de Xénope, éventuellement en présence d'un précurseur ou d'un oligonucléotide mutagène, pour récupérer, selon le cas, une matrice d'ADN ou d'ARN, éventuellement marquée de manière froide ou radio-active, ou un ADN recombinant ou un ADN superhélicoïdal, ou un ADN muté, ou une protéine codée par un gène déterminé.

Selon un mode de mise en oeuvre de ce procédé, appliqué à la synthèse d'ADN in-vitro, de l'ADN est incubé avec de l'extrait de cellules germinales de Xénope, à une température, à des concentrations et pendant une durée appropriées, pour récupérer une quantité significative d'ADN synthétisé.

Selon une modalité avantageuse de ce mode de mise en oeuvre, de l'ADN simple-brin est incubé avec de l'extrait de cellules germinales de Xénope, pendant une durée appropriée comprise de préférence entre 0 h 30 et 2 h, avantageusement à une température comprise entre 20˚ C et 37˚ C, à une concentration de l'ordre de 0,2 microgrammes à 2 milligrammes d'ADN par millilitre d'extrait, pour récupérer jusqu'à 5 microgrammes d'ADN synthétisé pour 100 micro-litres d'extrait.

Selon une autre modalité avantageuse de ce mode de mise en oeuvre, on incube de l'ADN simple-brin et un précurseur marqué approprié, avec de l'extrait de cellules germinales de Xénope, pendant une durée appropriée, de préférence comprise entre 0 h 30 et 2 h environ, à une température comprise entre 20˚ C et 37˚ C et à une concentration de l'ordre de 0.2 microgrammes à 2 milligrammes d'ADN simple-brin par ml d'extrait, pour obtenir une matrice d'un ADN marqué sous sa forme native.

Selon une disposition avantageuse de cette modalité, le précurseur marqué est un deoxynucléotidetriphosphate radiomarqué ($^{32}$P ou $^3$H notamment) judicieusement choisi.

Selon une autre modalité avantageuse de ce mode de mise en oeuvre, l'ADN mis en oeuvre pour synthétiser la matrice d'ADN sous forme native, est un ADN double-brin.

Selon une disposition avantageuse de cette modalité, l'ADN double-brin est préalablement incubé avec de la désoxyribonucléase avant d'être mis en présence de l'extrait de cellules germinales de Xénope. L'on obtient ainsi un ADN double-brin dont chacun des deux brins est marqué.

Selon un autre mode de mise en oeuvre du procédé conforme à la présente invention appliqué à la ligation trans d'ADN in vitro, des séquences d'ADN à rabouter sont incubées dans de l'extrait de cellules germinales de Xénope pendant une durée appropriée, avantageusement de l'ordre de 2 à 16 heures, à des températures appropriées, avantageusement comprises entre 12˚ C et 20˚ C et à une concentration de 0,2 à 5 microgrammes d'ADN par ml d'extrait, pour obtenir un ADN recombinant.

Selon un mode de mise en oeuvre du procédé conforme à la présente invention appliqué à la ligation cis d'ADN in vitro, de l'ADN circulaire double-brin contenant des coupures - ou nicks - est incubé avec de l'extrait de cellules germinales de Xénope pendant une durée appropriée, avantageusement comprise entre 4 h et 16 h environ, à la température ambiante, à une concentration pouvant aller jusqu'à 100 microgrammes d'ADN nické/ml d'extrait, pour obtenir un ADN ligaturé superhélicoïdal.

Selon encore un autre mode de mise en oeuvre du procédé conforme à la présente invention appliqué à la mutagénèse dirigée d'ADN in vitro, de l'ADN simple-brin est incubé avec un oligonucléotide mutagène, dans de l'extrait de cellules germinales de Xénope, pendant une durée appropriée, qui n'est généralement pas supérieure à 2 h, à la température ambiante, puis la réaction est arrêtée par addition de réactifs appropriés, l'ADN est récupéré et éventuellement purifié.

Selon un autre mode de mise en oeuvre du procédé conforme à la présente invention appliqué à la synthèse d'ARN in vitro, on fait incuber de l'ADN double-brin ou simple-brin dans de l'extrait de cellules germinales de Xénope, pendant environ deux heures à la température ambiante, à une concentration avantageusement de l'ordre de 1 à 100 microgrammes d'ADN par ml d'extrait, après quoi l'on extrait l'ARN synthétisé.

Bien entendu, l'on peut obtenir de l'ARN marqué, en introduisant dans le milieu réactionnel un précurseur marqué.

Selon encore un autre mode de mise en oeuvre du procédé conforme à la présente invention, appliqué à la synthèse de protéines in vitro, l'extrait de cellules germinales de Xénope est préalablement traité, au cours d'une première étape, par une nucléase, puis, au cours d'une deuxième étape, on ajoute de l'ARN exogène à cet extrait traité, après quoi l'on extrait la protéine recherchée par des méthodes appropriées.

Selon une modalité avantageuse de ce mode de mise en oeuvre, l'incubation de l'ARN exogène avec l'extrait traité de cellules germinales de Xénope, est effectuée pendant une à deux heures à la température ambiante, à une concentration de 10 à 40 microgrammes d'ARN par ml d'extrait.

Selon une autre modalité avantageuse de ce mode de mise en oeuvre, dans le cas où l'on cherche à synthétiser une protéine marquée, l'incubation est effectuée en présence d'un précurseur marqué approprié, tel qu'un aminoacide marqué, notamment.

Selon encore une autre modalité avantageuse de ce mode de mise en oeuvre, l'extrait de cellules germinales de Xénope est traité au cours de la première étape par la nucléase micrococcale de Staphylococcus aureus en présence de $CaCl_2$, puis l'action de la nucléase est stoppée, au bout de 15 minutes environ, par addition d'un inhibiteur approprié.

Selon une autre modalité avantageuse de ce mode de mise en oeuvre, l'extrait de cellules germinales de Xénope est traité au cours de la première étape par de la ribonucléase, avantageusement d'origine pancréatique, à une température comprise entre 8°C et 15°C, pendant environ 30 minutes au bout desquelles l'action de la ribonucléase est stoppée à l'aide d'un inhibiteur approprié.

Conformément à la présente invention, l'extrait de cellules germinales de Xénope mis en oeuvre est obtenu après centrifugation (s) des oeufs de Xénope, à basse vitesse (14 000 g éventuellement) précédée(s) d'une centrifugation lente (à 30-150 g) de brève durée.

Outre les dispositions qui précèdent, l'invention comprend encore d'autres dispositions, qui ressortiront de la description qui va suivre.

L'invention sera mieux comprise à l'aide du complément de description qui va suivre, qui se réfère à des exemples de préparation de l'extrait de cellules germinales de Xénope et d'application à différentes réactions fondamentales du domaine de la biologie moléculaire.

Il doit être bien entendu, toutefois, que ces exemples et les parties descriptives correspondantes, sont donnés uniquement à titre d'illustration de l'objet de l'invention, dont ils ne constituent en aucune manière une limitation.

## EXEMPLES

### EXEMPLE I

## FABRICATION DE L'EXTRAIT D'OEUFS DE XENOPE

### 1) Préparation des animaux

Les animaux sont élevés comme décrit par GURDON dans "METHODS IN DEVELOPMENTAL BIOLOGY, (1967), pp. 75-85. 150 unités d'hormone chorionique gonadotrope sont injectées dans les sacs lymphatiques dorsaux de xénopes femelles.

Huit heures plus tard une deuxième injection de 600 unités d'hormone est réalisée. Les animaux sont alors placés pendant une nuit dans des bacs contenant du HSB 1X (15 mM Tris pH 7,6, NaCl 110 mM, KCl 2mM, $MgSO_4$ 1mM, $Na_2HPO_4$ 0,5 mM).

### 2) Collecte et dégelification des oeufs

Les oeufs sont récoltés dans un bécher. Chaque femelle pond 10 000 à 15 000 oeufs. Les oeufs sont rincés dans l'eau du robinet, puis laissés dans l'eau distillée pendant environ 2 à 3 mn. Puis ils sont transférés dans une solution 2 % cystéine dans 0,8X Barth, pH 7,95. Une agitation lente permet de dissoudre la gelée entourant les oeufs. (Si la dissolution est difficile, on dilue la solution avec de l'eau distillée dans le bécher). Après dissolution de la gelée, les oeufs sont rincés quatre fois dans du Barth 0,8X puis triés sous la loupe binoculaire afin de retirer les oeufs éventuellement endommagés.

### 3) Obtention de l'extrait

Les étapes ultérieures sont réalisées à 0-1°C. Les oeufs sont déposés dans des tubes à centrifuger, à 0-1°C. Les oeufs sont rincés trois fois avec un tampon d'extraction présentant la composition suivante : Hépès 20mM, pH 7,6, acétate ou chlorure de potassium 70 mM, DTT 2mM, PMSF 1 mM, pepstatine 0.5 $\mu$M, leupeptine 0.5$\mu$M 5 % saccharose, $MgCl_2$ 2 mM, $CaCl_2$ 0,1mM. Puis on les laisse reposer pendant 2 mn et l'excès de tampon au-dessus des oeufs est totalement éliminé. Pour parfaire cette élimination du tampon extra-cellulaire, on peut procéder à une courte centrifugation des oeufs à basse vitesse (30 à 150 g, 40 à 60 sec.) sur un coussin d'huile neutre de densité intermédiaire entre l'oeuf et l'eau (densité 1.05 par exemple). Après centrifugation, les excès d'huile et de tampon sont éliminés. Les tubes contenant les oeufs sont alors centrifugés à 0-1°C, à 14 000 g pendant 20 mn, et l'extrait est collecté au-dessus du culot. Une deuxième centrifugation à 14 000 g, pendant 20 mn, est effectuée pour des volumes supérieurs à 1 ml. Une troisième centrifugation à 140000 g pendant 45 mn à 0-1°C, peut être effectuée dans un rotor à angle fixe SORVALL 50 TI ou un rotor à godets oscillants. Cette centrifugation permet de clarifier davantage l'extrait sans altérer ses capacités de replication de l'ADN simple-brin. Cette centrifugation ne doit pas être faite quand l'extrait doit être utilisé pour la synthèse de protéines. L'extrait final est congelé à - 80°C ou bien sous azote liquide par petites fractions. L'extrait ainsi obtenu est stable pendant une période d'au moins six mois.

On peut obtenir à partir de la ponte d'un seul animal, jusqu'à environ 10 ml d'extrait. L'extrait obtenu contient de façon active in vitro, tous les composants ioniques et non-ioniques nécessaires à la réalisation des réactions fondamentales de la biologie moléculaire et notamment une activité de replication de l'ADN simple-brin en ADN double-brin, couplée à une activité d'assemblage en chromatine de l'ADN en cours de replication (décrite dans ALMOUZNI et MECHALI, 1988, Loc. cité) ; des activités ligases ATP-dépendantes qui permettent de refermer des nicks qui se sont produits sur de l'ADN purifié à partir de plasmides ou de phages, etc...

4) Lyophilisation des extraits.

Les extraits peuvent être lyophilisés et gardés sous forme de lyophilisats. L'addition d'un volume d'eau distillée égal au volume de l'extrait avant lyophilisation permet de retrouver toute l'activité initiale de l'extrait. Ce procédé est particulièrement intéressant pour la conservation des extraits et leur application commerciale. Il permet également de concentrer l'extrait ; dans ce cas, on rajoute un volume d'eau inférieur au volume initial de l'extrait. La dissolution est immédiate.

EXEMPLE II

SYNTHESE D'ADN IN VITRO

A. Production de matrices radio-actives d'ADN $^{32}$P

Conformément à la présente invention, la seule addition d'ADN dans de l'extrait d'oeufs de Xenope tel qu'obtenu conformément à l'Exemple I, en présence d'un précurseur radio-actif, permet de fabriquer une matrice radio-active. Il s'agit de la seule méthode in-vitro connue à ce jour qui permette d'obtenir une matrice radio-active d'un ADN sous sa forme native.

Dans un microtube, on ajoute : 100 microlitres d'extrait + 1 microgramme d'ADN simple brin + 50 microcuries par ml d'ATP $\alpha$ $^{32}$P, 6000 curies par millimole (AMERSHAM). On incube à 20°C pendant 2h. On peut augmenter la vitesse de réaction en incubant à 37°C. Dans ces conditions, on obtient environ $15 \times 10^6$ cpm incorporés. On peut augmenter la quantité de radio-activité incorporée en augmentant la quantité d'ATP $^{32}$P dans le milieu, ou bien en dialysant au préalable l'extrait contre le tampon d'extraction décrit à l'Exemple I. La leupeptine et la pepstatine peuvent être ajoutées à l'extrait après la dialyse. On peut également inclure dans le tampon de dialyse le métabisulfite de Sodium 5 mM. Lors de l'utilisation de l'extrait, on ajoute dans le milieu réactionnel des déoxy- et des ribonucléotides froids à une concentration 0.2 mM, l'ATP 2 mM, $MgCl_2$ 6mM et le ou les nucléotide(s) radio-actif(s) à une concentration inférieure ou égale à 10 micromolaires dans le milieu d'incubation.

Les matrices d'ADN $^{32}$P obtenues de la sorte sont sous forme native et présentent une activité spécifique optimum de $10^8$ à $2 \times 10^9$ cpm par microgramme. Elles sont bien adaptées à l'hybridation moléculaire sur SOUTHERN ou NORTHERN BLOT, surtout si on les fragmente ultérieurement par de faibles concentrations en DNase ou par dépurination.

Ce type de protocole, comme les suivants, s'applique indifféremment à des volumes inférieurs ou supérieurs à 100 $\mu$l.

B. Production de matrices radio-actives d'ADN $^3$H

On procède comme décrit en A. ci-dessus, à ceci près que le précurseur radio-actif mis en oeuvre est le deoxynucleotide ³H.

Les matrices d'ADN ³H obtenues présentent une activité spécifique optimum de $5 \times 10^7$ cpm par microgramme.

## C. Production de matrices radio-actives à partir d'ADN double-brin

On incube au préalable l'ADN double-brin avec 2 nanogrammes/ml DNase I pendant 20 minutes à la température ambiante (20-22°C), puis on ajoute l'extrait et l'on poursuit comme décrit en A. ou B. ci-dessus.

## EXEMPLE III

### SYNTHESE DE MATRICES D'ADN FROIDES

Le processus est très proche de celui décrit à l'Exemple II ci-dessus, à ceci près qu'on ajoute uniquement l'ADN simple-brin à l'extrait d'oeufs de Xénope, à une concentration de 10 à 50 microgrammes d'ADN/ml d'extrait. On incube à 20°C pendant deux heures. On obtient 1 à 5 microgrammes d'ADN synthétisé pour 100 microlitres d'extrait.

## EXEMPLE IV

### PURIFICATION DES MATRICES APRES SYNTHESE

A 100 microlitres de milieu d'incubation contenant la matrice d'ADN synthétisée, on ajoute :
20 microlitres "Stop Mix" (EDTA 200 mM, SDS 5 %, pH 7,9)
6 microlitres Protéinase K, 10 mg/ml, pendant 30 mn à 1 heure, à 37°C
75 microlitres TNE (tampon Tris 15 mM, pH 7,8, NaCl 20 mM, EDTA 1 mM)
200 microlitres Phénol-Chloroforme.

On mélange vigoureusement pendant 20 à 30 secondes. Puis on centrifuge 5mn dans une centrifugeuse, à 14 000 g, à température ambiante. On prélève la phase aqueuse et on la met de côté. On réextrait la phase phénolique avec 100 microlitres TNE et on mélange la phase aqueuse avec la première. On réextrait les phases aqueuses (300 microlitres) avec 300 microlitres chloroforme-alcool isoamylique (24/1). On prélève la phase aqueuse et on ajoute 15 microlitres d'acétate de sodium 3M + 2 volumes éthanol 100 %. On laisse la solution à -20°C ou -80°C pendant 15 minutes à 1 heure, puis on centrifuge 15 mn à vitesse maximum dans une centrifugeuse à température ambiante. On reprend le culot dans le volume TNE désiré.

L'ADN ainsi obtenu est majoritairement sous forme bicaténaire hélicoïdale (ADN forme I). L'ADN peut ensuite être manipulé à volonté : utilisation comme matrice ADN natif, fragmentation par enzymes de restriction ou enzymes de modification, dénaturation, etc...

La synthèse du brin complémentaire de l'ADN permet d'obtenir un ADN super-hélicoïdal in-vitro avec une efficacité voisine de 100 %. C'est une réaction particulièrement intéressante lorsque l'on désire obtenir rapidement un ADN sous forme native in-vitro. Cette méthode est également très efficace lorsqu'on cherche à obtenir un ADN double-brin radio-actif sous forme native permettant d'étudier la chromatine ou l'interaction de facteurs spécifiques avec l'ADN. C'est la seule méthode in-vitro permettant d'obtenir ce type de réaction.

Lorsqu'une matrice radio-active est synthétisée à partir de l'ADN simple-brin, la matrice radio-active ainsi obtenue est asymétrique : un seul des deux brins de l'ADN est radio-actif. Cette propriété est extrêmement utile lorsqu'il est nécessaire de détecter une séquence spécifique d'un seul des deux brins de l'ADN, ou de rechercher un ARN transcrit de l'un ou l'autre brin de l'ADN, ou d'étudier l'interaction de facteurs spécifiques avec l'un ou l'autre brin.

## EXEMPLE V

### PRODUCTION DE SONDES RADIOACTIVES

Immédiatement avant son addition dans l'extrait, l'ADN est dénaturé à 100°C, pendant 10 mn, puis plongé dans de la glace ou un mélange alcool-glace jusqu'à refroidissement. On peut également dénaturer l'ADN par NaOH 0,3 M, 5 mn à 20-22°C, suivi de neutralisation par HCl 0,3 M. Puis la réaction en présence de l'extrait et du (des) déoxynucléotide(s) radioactif(s) est réalisée à une température de 20 à 37°C pendant 1 heure 30. L'ADN est ensuite purifié comme indiqué à l'Exemple IV

n peut obtenir des sondes encore plus radioactives par dialyse préalable ou filtration de l'extrait comme indiqué à l'Exemple X ci-après. Dans ce cas, on ajoute après dialyse les composants indiqués audit Exemple X.

La réaction peut également s'effectuer en ajoutant à l'ADN, avant dénaturation à 100°C ou immédiatement avant neutralisation par HCl, un mélange d'oligodéoxynucléotides (exemple héxanucléotides PHARMACIA) dans le rapport 2 à 8 µg d'oligonucléotides par 100ng d'ADN. Après refroidissement dans la glace ou neutralisation par l'HCl, on laisse le mélange 10 mn à 20°C avant addition de l'extrait. Si on utilise un extrait filtré ou dyalisé, il suffit d'ajouter uniquement l'ATP 2 mM, MgCl2 5mM, parmi les 4 ribonucléotides triphosphates. Les autres composants à ajouter sont comme indiqué précédemment.

## EXEMPLE VI

## LIGATION DE L'ADN IN VITRO

### A. Ligation en cis d'un ADN circulaire double-brin contenant des nicks

On incube l'ADN nické avec l'extrait d'oeufs de Xénope. à une concentration pouvant aller jusqu'à 100 microgrammes ml d'extrait. pendant quatre heures à 20°C. On peut laisser incuber la réaction pendant 16 heures sans dommage. On extrait l'ADN comme décrit à l'Exemple IV. L'ADN obtenu est ligaturé et super-hélicoïdal.

### B. Ligation en trans

Ce type de ligation est extrêmement utilisé pour cloner et isoler des séquences d'ADN. La ligation permet d'insérer la séquence désirée dans un vecteur bactérien ou eucaryote.

L'utilisation d'extrait d'oeufs de Xénope permet de réaliser la ligation en trans en une seule addition des séquences à rabouter dans l'extrait. La réaction se fait en incubant l'ADN à une concentration de 1 à 5 microgrammes d'ADN/ml d'extrait, à la température ambiante, pendant 2 h, ou 12 à 15°C pendant 2 h à 16 h. On extrait l'ADN recombinant comme décrit à l'Exemple IV. Les ligations effectuées dans l'extrait sont indifféremment à bouts francs (blunt-ended) ou à bouts cohésifs (cohesive ends).

## EXEMPLE VII

## MUTAGENESE DIRIGEE IN VITRO

Les extraits d'oeufs de Xénope constituent le premier système totalement eucaryote permettant de réaliser la mutagénèse dirigée in-vitro, et ce en une seule addition. De plus l'extrait permet de réaliser une mutagénèse dirigée in-vitro, avec une efficacité au moins 10 à 15 fois supérieure aux systèmes classiques utilisant la DNA polymérase de KLENOW et la DNA ligase. La réaction ne nécessite que l'ADN et l'oligonucléotide mutagène et s'effectue en l'espace d'une seule journée.

Cette technique utilise les différents constituants cellulaires présents dans l'extrait et maintenus actifs dans les différentes réactions nécessaires à l'obtention de l'ADN muté. alors que dans tous les systèmes connus à ce jour, l'obtention de l'ADN muté nécessite l'addition d'ADN polymérase purifiée de E. COLI, de la DNA ligase, des quatre déoxynucléotides-triphosphates. de l'ATP et d'un tampon contenant du Tris, du MgCl2, du NaCl et du 2-mercaptoéthanol.

L'ADN simple-brin (0,5 microgramme) est hybridé avec 5 picomoles du primer mutagène (17 à 30 mers) et ajouté dans 50 microlitres d'extrait pendant 2 h à 20-22°C. La réaction est stoppée par 30 mM EDTA, SDS 0,1 % et traitée à la protéinase K, 600 microgrammes/ml, pendant 1 h à 37°C. L'ADN est alors extrait par le phénol-chloroforme. puis précipité à l'éthanol. comme décrit à l'Exemple IV. L'ADN est repris dans 25 microlitres TNE. On peut purifier encore l'ADN par mini-colonnes de NACS Prepacs (BRL) ou GENE CLEAN (BIO 101) ou QIAGEN (MALLET) pour augmenter l'efficacité de transformation, mais sans cependant augmenter la proportion de mutants obtenus. Des aliquots de 0,1, 1 et 10 microlitres sont utilisés pour transformer des bactéries compétentes BMH 71-18 mut L, qui est une souche réparation déficiente (RADMAN et WAGNER ANN. REV. GENET. 1986, 20, 523-538). Les cellules compétentes sont préparées par la méthode de HANAHAN (DNA CLONING, A PRACTICAL APPROACH, 1985, VOL. I, p. 109-135). Les bactéries transformées sont mises à pousser sur un tapis de bactéries de type sauvage BMH71-18, et les mutants sont alors caractérisés.

## EXEMPLE VIII

## SYNTHESE D'ARN IN VITRO

L'ADN est ajouté dans l'extrait d'oeufs de Xénope à une concentration de 10 à 100 μg d'ADN/ml d'extrait, 0,5-1u/μl RNasine (PROMEGA ou BRL), et 10 à 50 microcuries de GTP $^{32}$P si l'on désire un produit radioactif. La réaction est incubée 1 à 2 heures à la température ambiante. La transcription peut être stimulée dans certains cas (selon le gène transcrit) par addition de 3mM ATP et 5mM MgCl2. La synthèse peut être effectuée à partir d'ADN simple-brin ou double-brin ajouté dans l'extrait. Dans le premier cas, la transcription est observée sur un ADN assemblé en chromatine pendant la replication. Dans le deuxième cas, la transcription est observée sur un ADN assemblé en chromatine en l'absence de replication. Ces systèmes peuvent ainsi servir de système modèle unique dans l'étude de la régulation génétique in vitro.

L'efficacité de transcription peut être différente dans les deux cas et reflète des régulations génétiques différentes selon que l'ADN est assemblé en chromatine pendant la replication ou en son absence. La transcription peut aussi être stimulée par retrait des histones. Ceci peut être effectué par addition d'ADN non spécifique (exemple ADN de phage lambda, 50-100 μg/ml) ou de tout autre composant se liant aux histones sans altérer la transcription. L'ARN est extrait à la fin de la réaction comme indiqué dans l'Exemple IV pour l'extraction de l'ADN. Au cas où l'ARN doit être séparé de l'ADN, le milieu d'incubation peut être traité selon la technique de AUFFRAY et ROUGEON (EUR. J. BIOCHEM 1980, 107, p. 303-314).

Lorsque l'on ajoute au mélange contenant l'ADN simple-brin du rGTP radioactif, on observe en plus de la synthèse d'ARN radioactif, l'incorporation de radioactivité dans l'ADN pendant sa replication. Cette réaction est due à un transfert de radioactivité du ribonucléotide radioactif vers un ou des déoxy ribonucléotides de l'extrait, qui sont ensuite incorporés dans l'ADN. Cette réaction indique que l'extrait est probablement actif dans la conversion des ribonucléotides en déoxyribonucléotides tri-phosphate.

## EXEMPLE XI

### SYNTHESE DE PROTEINES IN-VITRO

#### A. Méthode CaCl₂-EGTA

Un extrait , fait à 14 000 g, est traité au préalable par 150 à 1000 unités/ml de nucléase micrococcale de Staphylococcus aureus en présence d' 1 mM CaCl₂ pendant 15 mn. Puis on ajoute 10 mM EGTA, 2 mM ATP, et 3 mM MgCl₂. On peut également rajouter à l'extrait 30 μg/ml de tARN de foie de boeuf. L'extrait est gardé congelé à - 80°C. L'extrait peut également être filtré sur une colonne de Sephadex G-25 (PHARMACIA) ou de Biogel P (BIORAD) prééquilibrée avec le tampon Hépès 10 mM pH 7.6, 40 mM KCl, 10 mM NaCl, 1 mM MgCl₂, 0.2 mM EGTA, 2mM dithiothreitol, à 0-4°C. L'extrait filtré est congelé à -80°C. La dialyse peut être également utilisée à la place de la filtration pour éliminer les petites molécules. Dans ce cas, le tampon de colonne est utilisé comme tampon de dialyse et on ajoute après dialyse 0,5 μM pepstatine, 0,5 μM leupeptine et 0,25 u/ml RNasine. L'extrait est alors prêt pour l'addition d'ARN exogène (10 à 20 microgrammes/ml), de RNasine 0,5 u/μl et de précurseur radio-actif (³⁵S méthionine) si l'on souhaite synthétiser une protéine radio-active. La réaction est incubée 1 à 2 h à 20°C et les protéines sont caractérisées (Methods in Enzymology, Vol 152).

Lorsque l'extrait dialysé ou filtré est utilisé, on ajoute en plus de l'ARN exogène et de la méthionine ³⁵S, un mélange d'acides aminés froids à la concentration de 20 mM pour chacun des 20 acides aminés. Si on veut obtenir une protéine très radioactive, on omet la méthionine du mélange d'acides aminés. On ajoute également au milieu réactionnel un dixième du volume d'une solution ATP 10 mM, GTP 2 mM, créatine phosphate 80 mM, spermidine 5 mM, dithiothreitol 20 mM, hepes 200 mM pH 7.6, glucose 6 Phosphate 2 mM, créatine Kinase 500 μg/ml, RNasin 10 u/μl.

#### B. Méthode RNase-inhibiteur de RNase

L'extrait est traité par la RNase pancréatique (1 microgramme/ml) pendant 30m mn à 10°C. Puis on ajoute 600 unités/ml "RNasin" (PROMEGA ou BRL) ou 10 unités "Inhibit-ACE" (MALLET SA). L'extrait est alors prêt pour l'addition d'ARN exogène comme décrit en A ci-dessus.

## EXEMPLE X

### SEQUENCAGE DE L'ADN

Alors que les techniques actuelles de séquençage sont lourdes et coûteuses, car elles nécessitent l'addition de plusieurs composants et l'utilisation d'enzymes très onéreuses : DNA polymérase de Klenow, DNA polymérase de T₄, Sequenase, Taq polymérase, l'extrait conforme à l'invention est capable d'assurer la conversion d'un ADN simple-brin en double-brin sur des distances très longues et aux processivités inégalées. L'ADN simple-brin du phage M13, communément utilisé comme vecteur de clonage et de séquençage, est une des meilleures matrices de l'extrait puisqu'il est converti en forme double-brin avec une efficacité voisine de 100 % en mois de deux heures. De plus, la fidélité de replication dans l'extrait est largement supérieure à celle obtenue à l'aide d'enzymes purifiées (résultats personnels non publiés).

Il ressort des exemples qui précèdent que l'extrait de cellules germinales de Xénope qui constitue le système eucaryote multifonctionnel in-vitro conforme à la présente invention, permet de réaliser totalement in vitro. en un minimum d'étapes et avec un nombre réduit de réactifs, à la fois la synthèse de matrices d'ADN ou d'ARN, la ligation de l'ADN. la mutagénèse dirigée in vitro, la synthèse de protéines in vitro et le séquençage de l'ADN. La possibilité de réaliser in vitro des réactions multiples et différentes dans un extrait qui constitue un système unique efficace pour toutes ces réactions, sans modification aucune, est tout à fait nouvelle.

## Revendications

1. Nouveau système eucaryote multifonctionnel in vitro. apte à permettre à lui seul la réalisation in vitro d'importantes réactions utilisées en biologie moléculaire et en génie génétique, notamment la synthèse d'acides nucléiques, la synthèse de protéines. la ligation de l'ADN. la mutagénèse dirigée in vitro, lequel système eucaryote est caractérisé en ce qu'il est constitué par un extrait de cellules germinales de Xénope obtenu à la suite d'au moins une centrifugation à 0-1°C à 14 000 g pendant 20 minutes, éventuellement suivie(s) d'une centrifugation à 140 000 g pendant 45 minutes à 0-1°C et/ou éventuellement précédée d'une centrifugation de brève durée à faible vitesse de l'ordre de 30 à 150 g, pendant 40 à 60 secondes.

2. Procédé de production d'extraits d'oeufs de Xénope à partir d'oeufs récoltés résultant de la ponte de Xénopes femelles préalablement traitées par de l'hormone chorionique gonadotrope, caractérisé en ce que les oeufs préalablement dégélifiés et rincés par un tampon d'extraction approprié, sont soumis à au moins une centrifugation à 0-1°C, à 14 000 g pendant environ 20 minutes, à la suite de laquelle ou desquelles l'extrait est recueilli et stocké à basse température (congélation à -80°C ou sous azote liquide), cette ou ces centrifugation(s) étant éventuellement suivie(s) d'une centrifugation de l'extrait collecté au-dessus du culot de la ou des première(s) centrifugation(s), à 140 000 g pendant 45 minutes environ, à 0-1°C. et/ou étant éventuellement précédée(s) d'une centrifugation de brève durée à faible vitesse, de l'ordre de 30 à 150 g pendant 40 à 60 secondes, avantageusement dans une huile neutre. de densité intermédiaire entre l'oeuf et l'eau, destinée à éliminer le tampon d'extraction des oeufs.

3. Procédé de réalisation de plusieurs réactions fondamentales en biologie moléculaire à l'aide d'un seul et unique système multifonctionnel eucaryote, notamment de réalisation de la synthèse d'acides nucléiques, de la synthèse de protéines, de la ligation de l'ADN, de la mutagénèse dirigée in-vitro, caractérisé en ce qu'on incube un acide nucléique de départ avec de l'extrait de cellules germinales de Xénope selon la revendication 1, éventuellement en présence d'un précurseur ou d'un oligo-nucléotide mutagène, pour récupérer, selon le cas, une matrice d'ADN ou d'ARN, éventuellement marquée de manière froide ou radio-active, ou un ADN recombinant ou un ADN superhélicoïdal, ou un ADN muté, ou une protéine codée par un gène déterminé.

4. Procédé selon la revendication 3, appliqué à la synthèse d'ADN in vitro, caractérisé en ce que de l'ADN est incubé avec de l'extrait de cellules germinales de Xénope selon la revendication 1, à une température, à des concentrations et pendant une durée appropriées, pour récupérer une quantité significative d'ADN synthétisé.

5. Procédé selon la revendication 4, caractérisé en ce que de l'ADN simple-brin est incubé avec de l'extrait de cellules germinales de Xénope selon la revendication 1, pendant une durée appropriée comprise de préférence entre 0 h 30 et 2 h, avantageusement à une température comprise entre 20°C et 37°C. à une concentration de l'ordre de 0,2 microgrammes à 2 milligrammes d'ADN par millilitre d'extrait, pour récupérer jusqu'à 5 microgrammes d'ADN synthétisé pour 100 microlitres d'extrait.

6. Procédé selon la revendication 4, caractérisé en ce qu'on incube de l'ADN simple-brin et un précurseur marqué approprié, avec de l'extrait de cellules germinales de Xénope selon la revendication 1, pendant une durée appropriée, de préférence comprise entre 0 h 30 et 2 heures environ, à une température comprise entre 20°C et 37°C et à une concentration de l'ordre de 0,2 à 10 microgrammes d'ADN simple-brin pour 100 microlitres d'extrait, pour obtenir une matrice d'un ADN marqué, sous sa forme native.

7. Procédé selon la revendication 6, caractérisé en ce que le précurseur marqué est un précurseur radio-actif tel qu'un deoxynucléotide-triphosphate radiomarqué ($^{32}$P ou $^{3}$H) ou un marquer froid judicieusement choisi.

8. Procédé selon la revendication 4, caractérisé en ce que l'ADN mis en oeuvre pour synthétiser la matrice d'ADN sous forme native, est un ADN double-brin.

9. Procédé selon la revendication 8, caractérisé en ce que l'ADN double-brin est préalablement incubé avec de la désoxyribonucléase avant d'être mis en présence de l'extrait de cellules germinales de Xénope.

10. Procédé selon la revendication 3, appliqué à la ligation trans d'ADN in vitro, caractérisé en ce que des séquences d'ADN à rabouter sont incubées dans de l'extrait de cellules germinales de Xénope selon la revendication 1, pendant une durée appropriée, avantageusement de l'ordre de 2 à 16 heures, à des températures appropriées, avantageusement comprises entre 12° C et 20° C et à une concentration de l'ordre de 0.2 à 5 microgrammes d'ADN par ml d'extrait, pour obtenir un ADN recombinant.

11. Procédé selon la revendication 3, appliqué à la ligation cis d'ADN in vitro, caractérisé en ce que de l'ADN circulaire double-brin contenant des coupures - ou nicks - est incubé avec de l'extrait de cellules germinales de Xénope selon la revendication 1, pendant une durée appropriée, avantageusement comprise entre 4 h et 16 h environ, à la température ambiante, à une concentration pouvant aller jusqu'à 100 microgrammes d'ADN nické/ml d'extrait, pour obtenir un ADN ligaturé superhélicoïdal.

12. Procédé selon le revendication 3, appliqué à la mutagénèse dirigée d'ADN in vitro, caractérisé en ce que de l'ADN simple-brin est incubé avec un oligonucléotide mutagène, dans de l'extrait de cellules germinales de Xénope selon la revendication 1, pendant une durée appropriée, qui n'est généralement pas supérieure à 2 h, à la température ambiante, puis la réaction est arrêtée par addition de réactifs appropriés, l'ADN est récupéré et éventuellement purifié.

13. Procédé selon la revendication 3, appliqué à la synthèse d'ARN in vitro, caractérisé en ce qu'on fait incuber de l'ADN simple-brin ou double- brin dans de l'extrait de cellules germinales de Xénopes selon la revendication 1, pendant environ 1 à 2 heures à la température ambiante, à une concentration de l'ordre de 1 à 100 microgrammes d'ADN par ml d'extrait, après quoi l'on extrait l'ARN synthétisé.

14. Procédé selon la revendication 3, appliqué à la synthèse de protéines in vitro, caractérisé en ce que l'extrait de cellules germinales de Xénope selon la revendication 1 est préalablement traité, au cours d'une première étape, par une nucléase, puis, au cours d'une deuxième étape, on ajoute de l'ARN exogène à cet extrait traité, après quoi l'on extrait la protéine synthétisée.

15. Procédé selon la revendication 14, caractérisé en ce que l'extrait de cellules germinales de Xénope mis en oeuvre est obtenu, conformément à la revendication 2, par centrifugation d'oeufs de Xénope à faible vitesse, de l'ordre de 30 à 150 g pendant 40 à 60 secondes, suivie d'une à deux centrifugations à 14 000 g pendant environ 20 minutes, à 0-1° C environ.

16. Procédé selon la revendication 14, caractérisé en ce que l'incubation de l'ARN exogène avec l'extrait traité de cellules germinales de Xénope, est effectuée pendant une à deux heures à la température ambiante, à une concentration de 10 à 40 microgrammes d'ARN par ml d'extrait.

17. Procédé selon l'une quelconque des revendications 14 et 16, caractérisé en ce que dans le cas où l'on cherche à synthétiser une protéine marquée, l'incubation est effectuée en présence d'un précurseur marqué approprié, tel qu'un amino-acide marqué, notamment.

18. Procédé selon la revendication 14, caractérisé en ce que l'extrait de cellules germinales de Xénope est traité, au cours de la première étape par la nucléase micrococcale de Staphylococcus aureus, en présence de $CaCl_2$, puis l'action de la nucléase est stoppée, au bout de 15 minutes environ, par addition d'un inhibiteur approprié.

19. Procédé selon la revendication 14, caractérisé en ce que l'extrait de cellules germinales de Xénope est traité au cours de la première étape par de la ribonucléase, avantageusement d'origine pancréatique, à une température comprise entre 8° C et 15° C, pendant environ 30 minutes, au bout desquelles l'action de la ribonucléase est stoppée à l'aide d'un inhibiteur approprié.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| D,Y | CELL, vol. 47, 1986, pages 577-587; J.J. BLOW et al.: "Initiation of DNA replication in Nuclei and purified DNA by a cell-free extract of Xenopus eggs" <br> * En entier * <br> --- | 1-19 | C 12 P 19/34 <br> C 12 N 15/10 |
| Y | METHODS IN ENZYMOLOGY, vol. 101, 1983, pages 370-386; J.B. GURDON: "The use of Xenopus oocytes for the expression of cloned genes" <br> * En entier * <br> --- | 1-19 | |
| Y | NUCLEIC ACIDS RESEARCH, vol. 17, no. 3, 11 février 1988, pages 907-924; P. PFEIFFER et al.: "Joining of nonhomologous DNA double strand breaks in vitro" <br> * En entier * <br> --- | 1-19 | |
| Y | CHEMICAL ABSTRACTS, vol. 100, no. 21, 21 mars 1984, page 230, résumé no. 170054w, Columbus, Ohio, US; M. MECHALI et al.: "Xenopus eggs as a model-system for DNA replication in eukaryotes", & UCLA SYMP. MOL. CELL. BIOL. NEW SER. 1983, 10(MECH. DNA REPLICATION RECOMB.), 563-80 <br> * En entier * <br> --- -/- | 1-19 | **DOMAINES TECHNIQUES RECHERCHES (Int. Cl.5)** <br><br> C 12 P <br> C 12 N |

Le présent rapport a été établi pour toutes les revendications

| Lieu de la recherche | Date d'achèvement de la recherche | Examinateur |
|---|---|---|
| LA HAYE | 01-02-1990 | PULAZZINI A.F.R. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)

Office européen
des brevets

**RAPPORT DE RECHERCHE EUROPEENNE**

Numero de la demande

EP 89 40 2313

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y | CHEMICAL ABSTRACTS, vol. 90, no. 3, 15 janvier 1979, page 194, résumé no. 17784q, Columbus, Ohio, US; O. SCHMIDT et al.: "Specific transcription of eukaryotic tRNA genes in Xenopus germinal vesicle extracts", & PROC. NATL. ACAD. SCI. U.S.A. 1978, 75(10), 4819-23 * En entier * --- | 1-19 | |
| Y | CHEMICAL ABSTRACTS, vol. 106, no. 5, 2 février 1987, page 176, résumé no. 28918m, Columbus, Ohio, US; J. BLOW et al.: "Initiation of DNA replication in nuclei and purified DNA by a cell-free extract of Xenopus eggs", & CELL (CAMBRIDGE, MASS.) 1986, 47(4), 577-87 * En entier * --- | 1-19 | |
| P,X | PROC. NATL. ACAD. SCI, USA, vol. 86, juin 1989, pages 4425-4429; P. BROOKS et al.: "Mismatch repair involving localized DNA synthesis in extracts of Xenopus eggs" * En entier * --- | 11 | DOMAINES TECHNIQUES RECHERCHES (Int. Cl.5) |
| P,X | NUCLEIC ACIDS RESEARCH, vol. 16, no. 17, 12 septembre 1988, Geneviève Almouzni et al.: "Oligonucleotide site-directed mutagenesis in Xenopus egg extracts" * En entier * --- -/- | 12 | |

Le présent rapport a été établi pour toutes les revendications

| Lieu de la recherche | Date d'achèvement de la recherche | Examinateur |
|---|---|---|
| LA HAYE | 01-02-1990 | PULAZZINI A.F.R. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| D,Y | THE EMBO JOURNAL, vol. 7, no. 3, 1988, pages 665-672; Geneviève Almouzni et al.: "Assembly of spaced chromatin promoted by DNA synthesis in extracts form Xenopus eggs" * En entier * | 1-19 | |

DOMAINES TECHNIQUES RECHERCHES (Int. Cl.5)

Le présent rapport a été établi pour toutes les revendications

| Lieu de la recherche | Date d'achèvement de la recherche | Examinateur |
|---|---|---|
| LA HAYE | 01-02-1990 | PULAZZINI A.F.R. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.................................................................................................
& : membre de la même famille, document correspondant

EPO FORM 1503 03.82 (P0402)